(19) **Europäisches Patentamt European Patent Office Office européen des brevets**

(11) **EP 2 715 316 B1**

(12) **EUROPEAN PATENT SPECIFICATION**

(45) Date of publication and mention of the grant of the patent:
**13.05.2015 Bulletin 2015/20**

(21) Application number: **12725523.0**

(22) Date of filing: **23.05.2012**

(51) Int Cl.:
*G01N 21/21* (2006.01)    *G01N 21/23* (2006.01)

(86) International application number:
**PCT/IB2012/052573**

(87) International publication number:
**WO 2012/164441 (06.12.2012 Gazette 2012/49)**

(54) **HAIR TREATMENT DEVICE HAVING A LIGHT-BASED HAIR DETECTOR**

HAARBEHANDLUNGSVORRICHTUNG MIT LICHTBASIERTEM HAARDETEKTOR

DISPOSITIF POUR LE TRAITEMENT DES POILS COMPORTANT UN DÉTECTEUR DE POILS BASÉ SUR LA LUMIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2011 EP 11168115**

(43) Date of publication of application:
**09.04.2014 Bulletin 2014/15**

(73) Proprietor: **Koninklijke Philips N.V.
5656 AE Eindhoven (NL)**

(72) Inventors:
• **HEINRICH, Adrienne
NL-5656 AE Eindhoven (NL)**

• **VAN HEESCH, Franciscus Hendrikus
NL-5656 AE Eindhoven (NL)**
• **VARGHESE, Babu
NL-5656 AE Eindhoven (NL)**
• **UZUNBAJAKAVA, Natallia Eduardauna
NL-5656 AE Eindhoven (NL)**

(74) Representative: **Coops, Peter
Philips
Intellectual Property & Standards
P.O. Box 220
5600 AE Eindhoven (NL)**

(56) References cited:
**EP-A1- 1 128 160       WO-A1-2010/106480
US-A1- 2007 252 997    US-A1- 2010 063 491
US-A1- 2011 022 039**

**Description**

FIELD OF THE INVENTION

This invention relates to a hair treatment device comprising a light-based detector for detecting a hair near a skin surface, the light-based detector comprising a light source for emitting optical radiation of at least a first wavelength and with an incident polarization towards the skin surface, a light sensor for detecting light reflected at the skin surface, the light sensor being capable of separately detecting the reflected light with the incident polarization and with a different polarization, and for providing a PP value representing the detected light with the incident polarization and a CP value representing the detected light with the different polarization, and a processor, coupled to the light sensor and being operative to discriminate between the skin surface and the hair based on the CP and PP value.
This invention further relates to a method for detecting a hair near a skin surface, and to a computer program product for detecting a hair near a skin surface.

BACKGROUND OF THE INVENTION

A light-based detector as used in the hair treatment device mentioned in the opening paragraph is, e.g., known from the international patent application published as WO 2010/106480 A1. This patent application describes a shaving device with an optical hair detection unit. The hair detection unit makes use of two light sources for emitting light of two different wavelengths and one incident polarization. The light is focused on a position on the user's skin. An imaging unit detects the light that is scattered or reflected at the user's skin. When light is not reflected by skin cells but by a hair constituting a birefringent object, the polarization is changed. The amount of polarization change induced by the hair is wavelength dependent. The imaging unit separately detects reflected light with different polarization states and different wavelengths. The differences in polarization change in different wavelength channels are used to discriminate between hair and skin surface.
The main performance parameters of the hair detection unit are sensitivity and specificity. Sensitivity is a measure for the chance that a hair is actually detected. Specificity is important to discriminate between hairs and other structures, in particular skin. In order to further improve the known hair detection unit, a light-based detector with a higher sensitivity and specificity is desired. Additionally, due to the use of multiple wavelengths, the known hair detection unit is a rather complex device with a lot of optical elements that have to be perfectly aligned in order to obtain a well working detection unit.

OBJECT OF THE INVENTION

It is an object of the invention to provide a hair treatment device of the kind mentioned in the opening paragraph with a light-based detector for detecting a hair near a skin surface, wherein the light-based detector has an improved sensitivity and specificity.

SUMMARY OF THE INVENTION

According to a first aspect of the invention, this object is achieved by a hair treatment device of the kind mentioned in the opening paragraph, wherein the light-based detector comprises a processor which is coupled to the light sensor and which is operative to, based on a calibration procedure, scale the CP value and the PP value to respective dynamic ranges in order to obtain a scaled CP value and a scaled PP value respectively, to determine a Minimum Intensity Projection (MIP) value of the set of the CP value and the PP value by selecting the lowest value of the scaled CP value and the corresponding scaled PP value, and to discriminate between the skin surface and the hair based on the MIP value .
Due to an improved detection algorithm, hairs constituting birefringent structures can be detected with a high sensitivity and specificity. Non-birefringent materials preserve the original incident polarization state of the light. The light or measures a high PP (parallel polarized) value and a low CP (cross polarized) value. When the light is reflected by a hair constituting a birefringent object, the polarization changes and the light-based detector will measure a relatively low PP value (light with the unchanged incident polarization state) and a relatively high CP value (light with the changed and thus different polarization state).
If the CP and PP values are properly scaled, taking the minimum of both values returns a high value for the hairs and low value for the skin surface areas. Mostly, this is the case already for the CP value alone, however, in some instances when non-hair structures are involved, the CP value can be high and the PP value low. Therefore, the MIP approach renders a more robust discrimination between skin and hair.
Experiments have shown that the use of the MIP value is a very sensitive and highly specific measure for detecting hairs constituting birefringent objects. Other options such as determining the difference or absolute difference between the CP value and the PP value, or dividing the absolute difference by the sum of both values have shown to be less successful in discriminating between hair and skin. The improved detection algorithm has even proved to be so successful that the use of light with only one wavelength is sufficient. Although using an additional light source emitting light of a second wavelength might still improve the effectiveness of the light-based detector, it is not necessary. As a result the improved algorithm allows for a less complex, cheaper and more robust light-based detector.

The dynamic ranges of the CP and PP signals are dependent on various circumstances. For example, the dynamic ranges may vary depending on skin and hair type or on the closeness and angle of the light-based detector to the tested skin surface. The dynamic ranges also differ from device to device. Therefore, the CP and PP values are preferably scaled to respective predefined ranges before the ratio is calculated. The respective dynamic ranges are preferably determined based upon a calibration procedure involving a background measurement and a diffuse standard. The background measurement may, e.g., be done by focusing the light at a predetermined depth in water or at a part of skin of which it is known that it does not comprise birefringent material. The diffuse standard may, e.g., be a hair at a controlled depth and position or a strongly scattering diffuse media with known optical properties.

When the light-based detector is used for hair detection, it was shown to be advantageous to use a predefined dynamic range for the PP value that is approximately three times as wide as the predefined dynamic range for the CP value. For example all PP values are scaled to [0,300] and all CP values are scaled to [0,100]. With these dynamic ranges the CP and PP values for the diffuse standard are similar. For other light-based detectors, the optimal dynamic ranges may be different.

In another embodiment of the hair treatment device according to the invention, the processor is further operative to calculate a ratio of the scaled CP value and the scaled PP value and to discriminate between the skin surface and the hair further based on the calculated ratio. When the light is reflected by non-birefringent skin material, the original incident polarization state of the light is preserved. The light sensor measures a high PP (parallel polarized) value and a low CP (cross polarized) value. The resulting CP/PP ratio thus is very small. When the light is reflected by a hair constituting a birefringent object, the polarization changes and the light-based detector will measure a relatively low PP value (light with the unchanged incident polarization state) and a relatively high CP value (light with the changed and thus different polarization state). When the light is reflected by the hair, the CP/PP ratio is significantly increased. Experiments have shown that the use of CP/PP ratio is a very sensitive and highly specific measure for detecting hairs constituting birefringent objects.

In the example where all PP values are scaled to [0,300] and all CP values are scaled to [0,100], the CP/PP ratio was close to zero for skin and around 1 for hairs. As a result, the CP/PP ratio could be used as some sort of probability value.

The CP/PP ratio (either obtained after scaling the CP and PP value or not) is preferably processed using a discriminating function. The discriminating function preferably is an S-shaped function with a steep slope. The result of applying the discriminating function is that a sharper transition from birefringent to non-birefringent is obtained. Consequently, the contrast between non-birefringent skin surfaces and birefringent hairs will be enhanced. For example, a hyperbolic tangent of the CP/PP ratio or of a linear function of the CP/PP ratio may be used for the discriminating function.

In a preferred embodiment, the light-based detector further comprises a control unit for controlling the light source to emit the optical radiation at multiple positions near the skin surface and at each position detecting the reflected light using the light sensor, the processor further being operative to provide a MIP map indicating for each position the corresponding MIP value. The multiple positions may form a one-dimensional scan line. Multiple one-dimensional scan lines may together form a two-dimensional scan surface. It is to be noted that the MIP map is not necessarily a 2D graphical representation of the surface area with elevated MIP values at some locations. The MIP map preferably is a list or array with MIP values for the multiple positions.

In a preferred embodiment of the hair treatment device according to the invention, the processor is further operative to calculate a ratio map by determining for each position a ratio of the corresponding CP value to the PP value, to apply a gradient filter to the ratio map in order to obtain an edge probability map indicating for each position a probability of the presence of an edge of the hair, and to combine the edge probability map and the MIP map to generate a binary map indicating where hairs are expected. The gradient filter may, e.g., be a first order Gaussian derivative filter. Applying a suitable gradient filter to the ratio map leads to low values in areas with stable CP/PP ratios. The CP/PP ratio is, e.g., relatively stable at skin parts without any nearby hairs. At and close to transitions between birefringent hairs and non-birefringent skin surfaces, the gradient filter produces higher values. The result of applying the gradient filter to the ratio map thus is a new map indicating the positions with a higher probability of comprising an edge of a birefringent hair.

While the MIP map provides a reliable probability map indicating where to expect birefringent hairs and where not, the edge probability map shows where the edges of the birefringent hairs are found. The combination of the edge probability map results in an even more reliable distinction between birefringent hairs and non-birefringent skin material. Furthermore, the edge probability information may be used for determining the shape of a detected birefringent hair. This makes it possible to make a reliable distinction between different kinds of birefringent objects. When using the light-based detector according to the invention for detecting hairs, the proposed detection algorithm may be used to discriminate between hairs and air bubbles that may also be birefringent.

In a further embodiment of a hair treatment device according to the invention, the light source is arranged to further emit optical radiation of a second wavelength, the light sensor is arranged for providing separate CP and PP values for the first and the second wavelength, and the processor is further operative to combine the CP and PP values for the first and the second wavelength and to determine the MIP value based on the combined CP and PP values for the first and second wavelength. The combined CP and PP values may, e.g., be obtained by taking a difference between or a ratio of the respective values for the different wavelengths. Alternatively, the combined CP and PP values may be a ratio of the difference between and the sum of the respective values for the different wavelengths.

A further embodiment of a hair treatment device according to the invention further comprises a hair-cutting laser source and a further processor which is coupled to the light-based detector, wherein the further processor is arranged to activate the hair-cutting laser source in a position near the skin surface in which the light-based detector has detected the presence of a hair. In this embodiment, the hair treatment device is a so-called laser-based shaving device. Other hair-treatment devices are also within the scope of the invention, for example light-based epilation devices wherein light energy is used to permanently or temporarily damage hair roots in order to obtain permanent or temporary hair removal, or hair coloring devices. Generally, the invention covers any hair treatment wherein a light-based detector for detecting the presence of hairs near the skin surface is or could be used.

The invention further relates to a method for detecting a hair near a skin surface, the method comprising the steps of emitting optical radiation of at least a first wavelength and with an incident polarization towards the skin surface, detecting light reflected at the skin surface and having the incident polarization and providing a PP value representing the detected light with the incident polarization, detecting light reflected at the skin surface and having a different polarization and providing a CP value representing the detected light with the different polarization, based on a calibration procedure, scale the CP value and the PP value to respective dynamic ranges in order to obtain a scaled CP value and a scaled PP value respectively, determine a Minimum Intensity Projection (MIP) value of the set of the CP value and the PP value by selecting the lowest value of the corresponding scaled CP value and the scaled PP value, and discriminate between the skin surface and the hair based on the MIP value.

The invention further relates to a computer program product for detecting a hair near a skin surface, which program is operative to cause a processor to perform the method for detecting a hair near a skin surface according to the present invention.

## BRIEF DESCRIPTION OF THE DRAWINGS

In the drawings:

Figure 1 schematically shows a light-based detector for detecting hairs constituting birefringent objects for use in a hair treatment device according to the invention,
Figure 2 shows a flow diagram of a method of detecting a hair as a birefringent object according to the invention,
Figure 3 schematically shows a light-based detector for detecting hairs constituting birefringent objects using two different wavelengths in a hair treatment device according to the invention,
Figures 4-6 show images illustrating different stages in a hair detection algorithm according to the invention, and
Figure 7 shows an exemplary hair treatment device according to the invention.

## DETAILED DESCRIPTION OF THE INVENTION

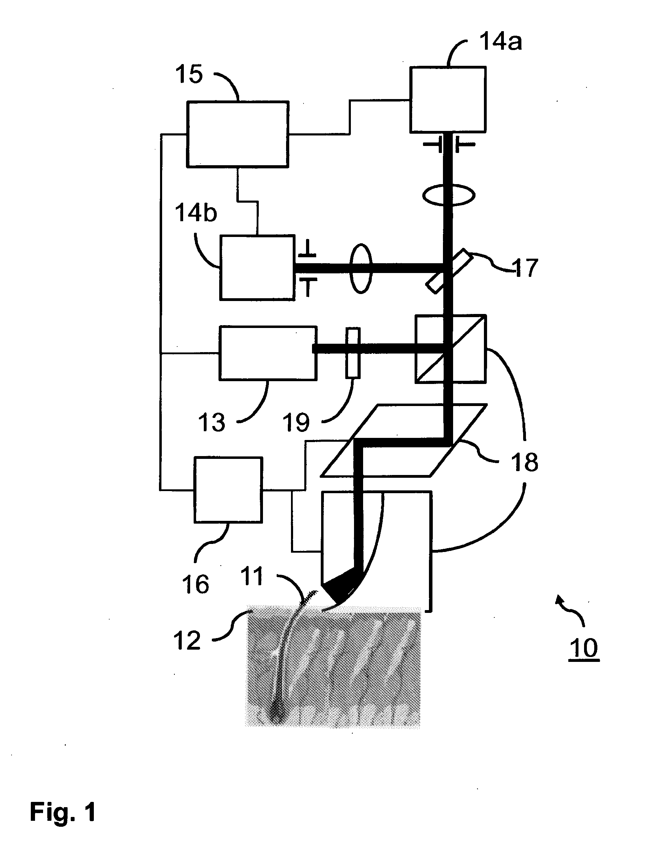

Figure 1 schematically shows a light-based detector 10 for detecting hairs constituting birefringent objects for use in a hair treatment device according to the current invention. The light-based detector 10 is adapted to detect hairs 11 on human or animal skin 12. Hair detection may be useful in IPL (Intense Pulsed Light) based or laser based hair cutting (shaving) or hair removal apparatuses.

The light-based detector 10 of figure 1 comprises a laser source 13 for emitting a laser beam, preferably in the near-infrared or infrared part of the spectrum. For example, light with a wavelength of 785 or 850 nm may be used. A polarizer 19 is used to provide a light beam with a well-defined incident polarization state. The incident polarization provide a light beam with a well-defined incident polarization state. The incident polarization state may be a linear or elliptical polarization state. Optical elements 18 focus the polarized light beam on the skin 12. A control unit 16 coupled to the laser source 13 and/or (part of) the optical elements 18 controls the exact optical path of the laser beam in order to control the exact position on the skin 12 that is tested for the presence of a birefringent hair 11 and to enable scanning lines or 2D areas of skin 12.

When the light is reflected at the skin surface 12, the reflected light preserves its original incident polarization state. Light interaction with birefringent hairs changes the polarization state of the light. As a result, the light reflected at a skin/hair sample may be partly parallel polarized (PP) with the incident beam and partly cross polarized (CP). The CP component of the reflected light is a measure for the probability that the light has hit upon a birefringent hair. In the light-based detector 10 of figure 1, the optical elements 18 lead the light reflected at the skin surface 12 to a polarization splitter 17. The polarization splitter 17 may e.g. use a Faraday rotator. The polarization splitter 18 splits the reflected beam into a PP beam which is subsequently focused on a first light sensor 14a and a CP beam which is focused on a second light sensor 14b.

A processor 15 is coupled to the light sensors 14a, 14b to receive the CP and PP signals and to use those signals for discriminating between hair and skin. The processor 15 may also be coupled to the laser source 13 and the control unit 16 for controlling the incident laser beam and scanning the skin surface 12.

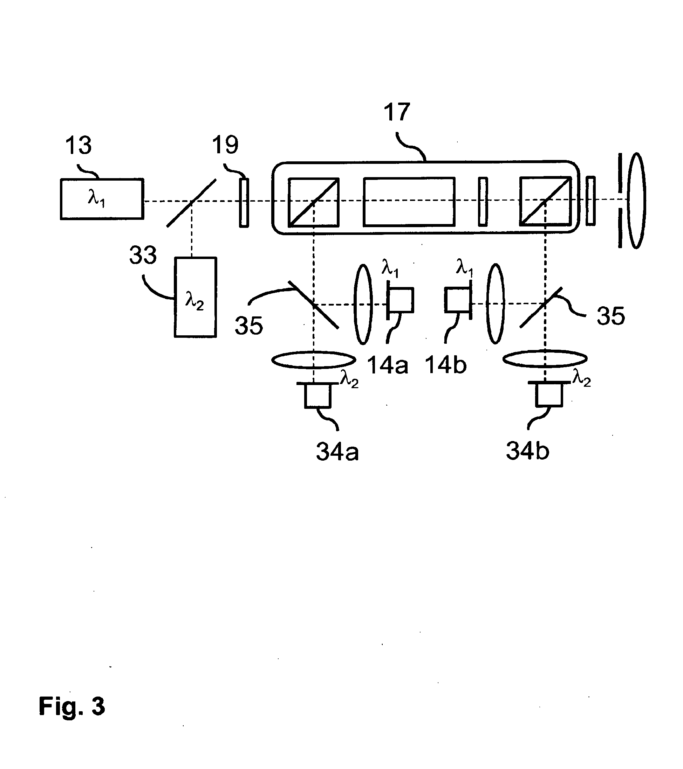

Optionally (see figure 3), an additional second laser source emitting light at a different wavelength is used. Like in WO 2010/106480 the differences in polarization change in different wavelength channels may be used to discriminate between hair and skin surface. When multiple laser sources and different wavelengths are used, additional light sensors are needed for separately detecting the reflected light at different wavelengths. Additional optical elements are needed for combining, splitting and focusing the different light beams (different wavelengths and/or different polarization states) towards the skin surface and the appropriate light sensors. The additional optical elements may include dichroic beam splitters for splitting a multi-color light beams into separate single-color light beams.

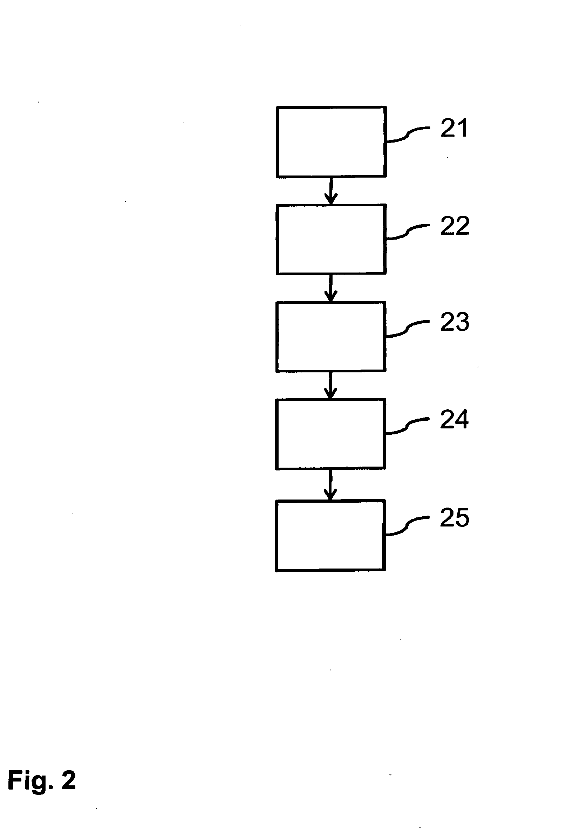

Figure 2 shows a flow diagram of the method of detecting a birefringent hair according to the invention. The method starts with a light emitting step 21. In this step the light source 13 emits light of at least a first wavelength and the polarizer 19 causes the light to have a well-defined incident polarization state. Under influence of the control unit 16, the optical elements 18 focus the emitted light beam onto a selected position on the skin surface 12.

When the emitted light reaches the skin 12, it is partially reflected back into the light-based detector 10. The optical elements 18 lead the reflected light towards the polarization splitter 17 where it is split into a PP and a CP signal. In PP detection step 22, the first light sensor 14a detects the light with the unchanged (PP) polarization and provides a PP value representing the detected light with the incident polarization. In CP detection step 23 the second light sensor 14b detects the light with the changed (CP) polarization and provides a CP value representing the detected light with the different polarization.

The light sensors 14a and 14b are coupled to the processor 15, which processes the obtained PP and CP values in order to detect birefringent hairs. The processing of the PP and CP values at least comprises selecting the lowest value of the CP and PP values as a Minimum Intensity Projection (MIP) value in MIP step 24. Additionally, a ratio of the CP value to the PP value may be calculated. Experiments have shown that the use of CP/PP ratio is a very sensitive and highly specific measure for detecting birefringent hairs 11. Other options such as determining the difference or absolute difference between the CP value and the PP value, or dividing the absolute difference by the sum of both values have shown to be less successful in discriminating between hair 11 and skin 12.

In a discriminating step 25 the processor 15 decides whether the light beam was reflected at a birefringent hair or not. The decision may be a binary yes/no decision or may result in a probability (typically a value between 0 and 1) of finding a birefringent hair at the tested position. If the light-based detector 10 only makes a measurement at one skin 12 position, the decision may be based upon the MIP value of the tested position only, e.g. by comparing the MIP value to a threshold value. In practice, the light-based detector 10 will scan an area of skin 12. The processor 15 preferably also takes the MIP values of nearby positions into account when determining the probability of finding a birefringent hair at the tested position.

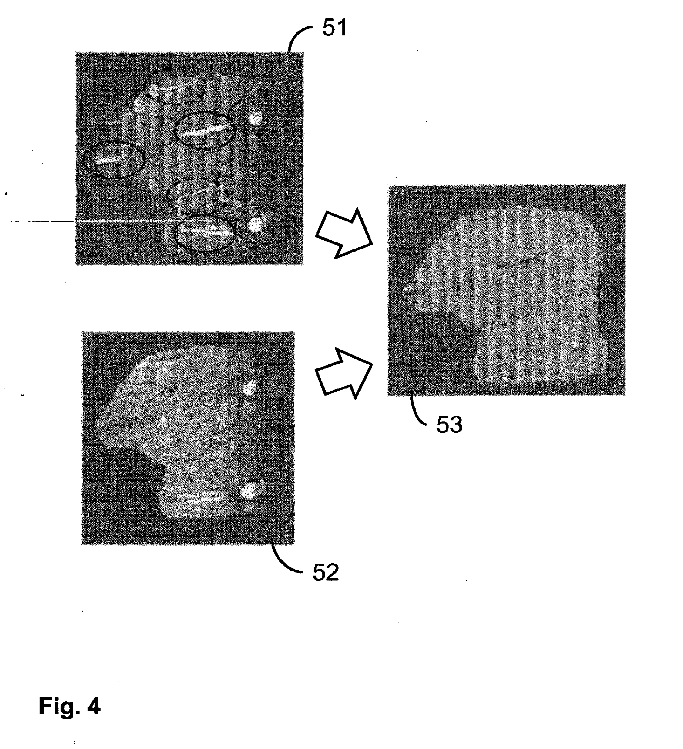

In addition, the CP/PP ratio may be used as the output value for the probability of finding a birefringent hair at the tested position. A threshold value may be used to make a binary yes/no decision for discriminating between hair 11 and skin 12. In a more advanced version of the discriminating algorithm, also other data processing operations are performed on the obtained CP and PP signals. Examples of such further data processing operations are illustrated by the images in figures 4-6. In the following some examples of (parts of) hair detection algorithms are described for detecting hairs in a hair/skin sample. Where detailed algorithms and exact numerical parameters are used, it should be noted that both the functions and the parameters may be chosen differently in different situations. For example, when looking for other types of birefringent objects, at least the parameters should be changed. However, the core principles behind the detection methods described below may also be used in other situations where the discrimination between birefringent and non-birefringent material is desired.

Figure 3 schematically shows a light-based detector for detecting hairs constituting birefringent objects using two different wavelengths. For this purpose, a second light source 33 is provided for emitting light at a different wavelength. The light-based detector may, e.g., use wavelengths of 785 and 850 nm. Higher contrast is obtained when using wavelengths that are less close to each other. For example, 785 and 1400 nm light may be used. Additional light sensors 34a, 34b are provided for measuring separate CP and PP values for the first and the second wavelength. The processor is operative to combine the CP and PP values for the first and the second wavelength and to determine the MIP value based on the combined CP and PP values for the first and second wavelength. The combined CP and PP values may, e.g., be obtained by taking a difference between $(CP[\lambda_1] - CP[\lambda_2], PP[\lambda_1] - PP[\lambda_2])$ or a ratio of $(CP[\lambda_1] / CP[\lambda_2], PP[\lambda_1]$

/ PP[$\lambda_2$]) the respective values for the different wavelengths. Alternatively, the combined CP and PP values may be a ratio of the difference between and the sum of the respective values for the different wavelengths ((CP[$\lambda_1$] - CP[$\lambda_2$]) / (CP[$\lambda_1$] + CP[$\lambda_2$]), (PP[$\lambda_1$] - PP[$\lambda_2$]) / (PP[$\lambda_1$] + PP[$\lambda_2$])), In the embodiment of figure 3, a Faraday rotator 17 is used for splitting the light of different polarizations and diverting the light to respective CP and PP channels. Dichroic filters 35 are used to split the light of different wavelengths, such that separate light sensors 14a/b, 34a/b can detect the CP and PP values for the different wavelengths.

Figures 4-6 show images illustrating different stages in a hair detection algorithm according to the invention. It is to be noted that the images shown have a purely illustrative function. For the light-based detector 10, only the values represented by the images are important. Although the light-based detector 10 might have a display for showing some of the images, this is certainly not necessary. In a hair-cutting (shaving) device, for example, the probability values can be used for deciding when to remove a hair and there is no need to visualize such probability values.

In figure 4, the images 51, 52 on the left show the CP values (top image 51) and the PP values (bottom image 52) obtained with a scan of a skin area comprising three hairs. The CP values 51 are low for normal non-birefringent skin parts and high for the birefringent hairs. The PP values 52 are high for normal non-birefringent skin parts and low for the birefringent hairs.

The dynamic range of the signals from the light sensors 14a, 14b can vary depending on skin and hair type and e.g. the closeness and angle of the blade with respect to the skin and hairs. Furthermore the sensor signals can be polluted with sensor noise. In order to be able to cope with a large dynamic range the signal is digitized and preconditioned to a predefined dynamic range. This may be done by controlling amplifiers that are between the light sensors 14a, 14b and the processor 15 or by the processor 15 itself. The scaling may be based on, e.g., the running average, recent minimum and/or recent maximum of the detected values. In the following examples, the CP values are scaled to a range [0,100]. Skin shows low intensity signals (e.g. 42) and hair high intensity signals (e.g. 99). The PP values are scaled to a range of [0,300]. Skin is characterized by high intensities (e.g. 175) and hair by lower intensities (e.g. 95). Other predefined ranges may be more useful in other situations, for example when looking for other types of birefringent objects.

In the CP image 51 several birefringent areas are encircled. Three of those areas (encircled by solid lines) comprise a hair. Two areas comprise an air bubble (encircled by dotted lines). Two other areas (also encircled by dotted lines) comprise other non-hair structures. In the PP image 52 only one of the hairs is clearly visible, while the other two are not. Also the bubbles are clearly visible. According to the invention, the CP values 51 and the PP values 52 are used to calculate CP/PP ratios for all tested points in the scanned area. An advantage of using the above described exemplary ranges for scaling the CP and PP values is that for hair, the CP value and the PP value do not differ too much and the CP/PP ratio for hair is close to 1. For skin areas, the CP value is low and the PP value is high, resulting in a very low CP/PP ratio. The high contrast between skin and hair that is obtained in this way improves the sensitivity of the light-based detector.

In the CP/PP image 53 in figure 4 the three hairs are clearly visible and also the two non-hair structures are visible. However, the bubbles shown in the CP image 51 and the PP image 52 have disappeared. The CP/PP image 53 thus shows an improved specificity over the original CP image 51 or PP image 52.

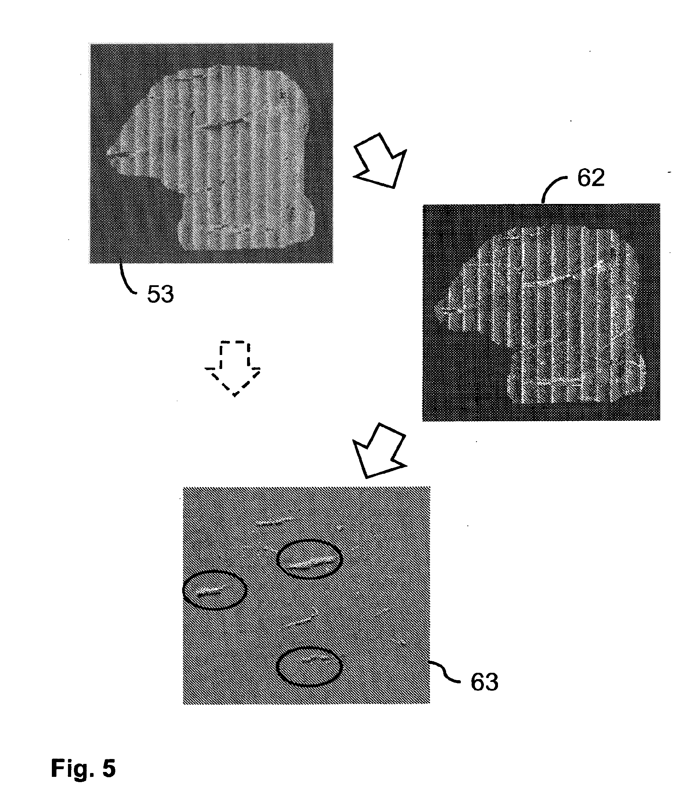

In figure 5, two steps for further processing the CP/PP ratio values 53 are illustrated. First, a discriminating function is used for further improving the contrast between birefringent and non-birefringent material. The discriminating function applied to the CP/PP ratio is an S-shaped function with a steep slope between CP/PP ratio values representing the transition between birefringent and non-birefringent material (in this example, between about 0.5 and about 0.8). For example, the following function may be used:

$$50 \left(1 + \tanh(3\,r - 2)\right), \text{ for } 0 < r < 0.8$$

$$50 \left(1 + \tanh(3\,r - 0.4)\right), \text{ for } 0.8 < r < 3$$

0, for 0 > 3

where r is the ratio value CP/PP.

An effect of using such a discriminating function with a steep slope is that a sharper transition from birefringent to non-birefringent material is obtained. Consequently, the contrast between non-birefringent skin surfaces and birefringent hairs will be enhanced. The second image 62 shows the result of applying the discriminating function to the CP/PP ratio values 53.

After applying the discriminating function, a gradient filter may be applied to the processed CP/PP data 62. A gradient filter determines a change of the CP/PP ratio with respect to position. For each tested position, a gradient filter provides an output that depends on the rate of change of the CP/PP ratio compared to the CP/PP ratio of nearby positions.

At the edges of birefringent hairs, the rate of change is high. Further away from the edges, the rate of change is low.

The output values of an exemplary gradient filter are used for obtaining the date shown in the third image 63 of figure 5. This example is obtained by applying a first order Gaussian derivative filter to the processed CP/PP ratio data of the second image 62. The result is an edge probability map 63, indicating for each position the chance that it comprises an edge of a birefringent hair. Due to the gradient processing, the two hair edges of a hair have opposite signs.

A further filtering step, e.g. using a finite impulse response (FIR) filter, may be performed for ensuring high intensity continuity at the edges and to remove non-hair outliers such as very narrow elongated structures (e.g. vellus hairs). To obtain the edge probability map 63 of figure 5, a 10-tap filter is used to cover a stretch of 40 $\mu$m. This ensures that both thin elongated non-hair structures and vellus hairs are not falsely detected. In a further optional data processing step by all the absolute values larger than a chosen $g_c$ are set to 100 and the remaining values are scaled to the range [-100,100]. The resulting edge probability map 63 is shown in figure 5. In this edge probability map 63 the three hairs (encircled) are clearly visible.

It is to be noted that, although the gradient filter in this example is applied to the processed CP/PP data 62 of the second image in figure 5, it is also possible to apply the gradient filter to the CP/PP ratio data 53 directly.

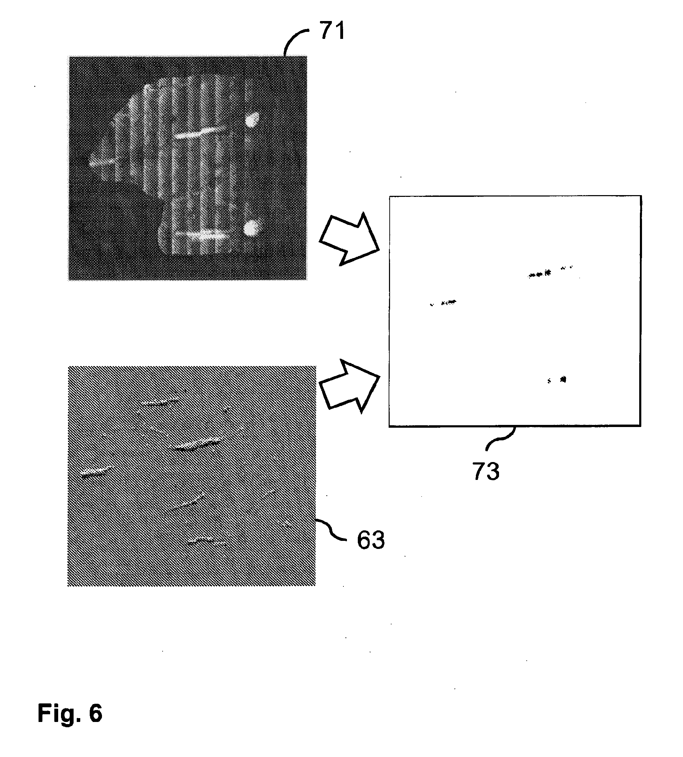

Figure 6 shows a further example of how the light-based detector data may be processed into a different useful form. In the pre-processed CP data 51 (assuming that the intensities are scaled to [0,100]), skin shows low intensity signals (e.g. 42) and hair high intensity signals (e.g. 99). In the pre-processed PP data 52 (with an intensity range of [0,300]), skin is characterized by high intensities (e.g. 175) and hair by lower intensities (e.g. 95). Taking the minimum of each data set per pixel (also referred to as Minimum Intensity Projection or MIP) returns a high intensity value for the hairs and low intensity value for the skin areas. Mostly, this is the case already for the CP data 51 alone, however, in some instances when non-hair structures are involved, the CP data 51 can be high and the PP data 52 low (e.g. 72 vs. 29). Therefore, the MIP approach renders a more robust discrimination between skin and hair. The result of taking the minimum of the CP and the PP data is shown in the top image 71 in figure 6. Like before, a FIR filter may be used for post-processing the MIP data 71, ensuring high intensity continuity and reducing sensor noise. Compared to the CP/PP ratio approach of figure 5, it is clear that this 'MIP map' 71 is useful for getting rid of the non-hair structures, but that it is less successful in discriminating between hairs and air bubbles.

In a preferred implementation of the algorithms according to the invention, the MIP data 71 and the edge probability map 63 are combined to make a final decision about where the hairs are found in the scanned skin/hair sample. As discussed above the CP/PP ratio approach resulting in the edge probability map is very effective in discriminating between hairs and air bubbles, while the MIP approach effectively discriminates between hairs and other non-hair structures. Combining both approaches may result in a hair detection algorithm with superior sensitivity and specificity. An algorithm for combining both approaches should look for points with a high probability of comprising a hair (= high value in MIP data 71) and with a high probability of finding hair edges at nearby positions (= high values in edge probability map 63 for nearby points). An exemplary algorithm for making such a combination is:

$$h(n) = 1, \text{ if } 0.7 \max(p_g[n - 20, \ldots, n]) + 0.3 \; p_m[n] > d_r,$$

and

$$h(n) = 0, \text{ else,}$$

wherein $p_g[n]$ refers to the edge probability value, $p_m[n]$ to the MIP value and $h(n) = 1$ to a detected hair at location $n$. $d_r$ is an experimentally determined threshold value. This algorithm searches for hair positions with a first hair edge somewhere in the previous 20 points (80 $\mu$m, if each scanning step takes 4 $\mu$m). The algorithm may be adapted for also taking into account the second hair edge:

$$h(n) = 1, \text{ if } 0.3 \max(p_g[n-20,\ldots,n]) + 0.5 \; p_m[n] - 0.2 \min(p_g[n,\ldots,n+20]) > d_e$$

$$h(n) = 0, \text{ else,}$$

wherein $p_g[n]$ refers to the edge probability value, $p_m[n]$ to the MIP value and $h(n) = 1$ to a detected hair at location $n$. $d_r$ is an experimentally determined threshold value. The binary image 73 on the right side of figure 6 shows the outcome of the combination of the MIP map 71 and the edge probability map 63 of figure 4. Here, there are clearly three hairs and no air bubbles or other non-hair structures visible. This combined approach thus results in a highly sensitive and specific hair detection algorithm.

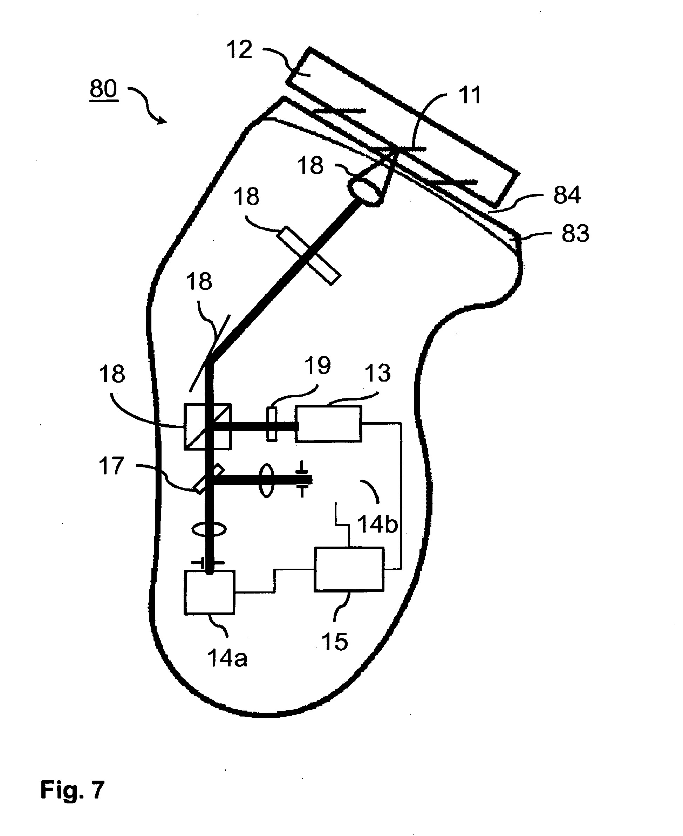

Figure 7 shows an exemplary hair treatment device according to the invention. The hair treatment device shown is a laser-based hair-cutting (shaving) device 80 for cutting or shortening hairs. The hair-cutting device 80 comprises a light-based detector for detecting hairs constituting birefringent objects similar to the one described above with reference to figure 1. Equal reference numbers correspond to similar features. In addition to features already discussed above, the hair-cutting device 80 may also comprise an optical or contact window 83 and may apply an immersion fluid 84. For example, the fluid 84 may be an index-matching fluid, having an index of refraction which is halfway between that of the optical window 83 and that of the skin 12. Preferably, all refractive indices are substantially equal. This also lowers the reflection from the skin 12. The fluid 84 may also be selected for the purpose of cooling the skin 12, or treating it otherwise. Furthermore, although the contact window 83 is optional, it helps in serving as a reference for determining the positions of the hairs 11.

The hair-cutting device 80 may not only use the laser source 13 for detecting the hair 11, but also for cutting it. When the laser source 13 is used for cutting, the laser source 13 may operate at a different power level than when detecting hairs. Alternatively, a separate laser source (not shown) may be used for the cutting of the hairs 11.

The control over the hair-detection and cutting process may be performed by the processor 15 or by a further processor (not shown) which is coupled to the light-based detector and to the hair-cutting laser source. The processor 15 or the further processor is arranged to activate the hair-cutting laser in positions near the skin surface in which the light-based detector detects or has detected the presence of a hair. For this purpose the detection and hair-cutting laser beams may be scanned over the skin surface. The skilled person will be able to construct suitable scanning means for this purpose, which are for example known from WO 00/062700, WO 2005/099607, or WO 2010/143108.

It will be appreciated that the invention also extends to computer programs, particularly computer programs on or in a carrier, adapted for putting the invention into practice. The program may be in the form of source code, object code, a code intermediate source and object code such as partially compiled form, or in any other form suitable for use in the implementation of the method according to the invention. It will also be appreciated that such a program may have many different architectural designs. For example, a program code implementing the functionality of the method or system according to the invention may be subdivided into one or more subroutines. Many different ways to distribute the functionality among these subroutines will be apparent to the skilled person. The subroutines may be stored together in one executable file to form a self-contained program. Such an executable file may comprise computer executable instructions, for example processor instructions and/or interpreter instructions (e.g. Java interpreter instructions). Alternatively, one or more or all of the subroutines may be stored in at least one external library file and linked with a main program either statically or dynamically, e.g. at run-time. The main program contains at least one call to at least one of the subroutines. Also, the subroutines may comprise function calls to each other. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth. These instructions may be subdivided into subroutines and/or be stored in one or more files that may be linked statically or dynamically.

The carrier of a computer program may be any entity or device capable of carrying the program. For example, the carrier may include a storage medium, such as a ROM, for example a CD ROM or a semiconductor ROM, or a magnetic recording medium, for example a floppy disc or hard disk. Further the carrier may be a transmissible carrier such as an electrical or optical signal, which may be conveyed via electrical or optical cable or by radio or other means. When the program is embodied in such a signal, the carrier may be constituted by such cable or other device or means. Alternatively, the carrier may be an integrated circuit in which the program is embedded, the integrated circuit being adapted for performing, or for use in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware.

**Claims**

1. A hair treatment device comprising a light-based detector (10) for detecting a hair near a skin surface, the light-based detector comprising:

   - a light source (13) for emitting optical radiation of at least a first wavelength and with an incident polarization towards the skin surface,
   - a light sensor (14a, 14b) for detecting light reflected at the skin surface, the light sensor being capable of separately detecting the reflected light with the incident polarization and with a different polarization, and for providing a PP value representing the detected light with the incident polarization and a CP value representing the detected light with the different polarization, and
   - a processor (15), coupled to the light sensor and being operative to

     - based on a calibration procedure, scale the CP value and the PP value to respective dynamic ranges in order to obtain a scaled CP value and a scaled PP value respectively,
     - determine a Minimum Intensity Projection (MIP) value of the set of the CP value and the PP value by selecting the lowest value of the scaled CP value and the corresponding scaled PP value, and discriminate between the skin surface and the hair based on the MIP value.

2. A hair treatment device as claimed in claim 1, wherein the respective dynamic ranges are determined based upon a calibration procedure involving a background measurement and a diffuse standard.

3. A hair treatment device as claimed in claim 1, wherein the dynamic range for the PP value is approximately three times as wide as the dynamic range for the CP value.

4. A hair treatment device as claimed in claim 1, wherein the processor is further operative to calculate a ratio of the scaled CP value and the scaled PP value and to discriminate between the skin surface and the hair further based on the calculated ratio.

5. A hair treatment device as claimed in claim 4, wherein the processor is further operative to use a discriminating function for further processing the calculated ratio, the discriminating function being an S-shaped function with a steep slope.

6. A hair treatment device as claimed in claim 5, wherein the discriminating function comprises a hyperbolic tangent of the calculated ratio or of a linear function of the calculated ratio.

7. A hair treatment device as claimed in claim 1, wherein the light-based detector further comprises a control unit for controlling the light source to emit the optical radiation at multiple positions near the skin surface and at each position detecting the reflected light using the light sensor, the processor further being operative to provide a MIP map indicating for each position the corresponding MIP value.

8. A hair treatment device as claimed in claim 7, wherein the multiple positions form a one-dimensional scan line.

9. A hair treatment device as claimed in claim 8, wherein multiple one dimensional scan lines together form a 2D scan surface.

10. A hair treatment device as claimed in claim 7, the processor further being operative to

    - calculate a ratio map by determining for each position a ratio of the corresponding CP value to the PP value,
    - to apply a gradient filter to the ratio map in order to obtain an edge probability map indicating for each position a probability of the presence of an edge of the hair, and to
    - combine the edge probability map and the MIP map to generate a binary map indicating where hairs are expected.

11. A hair treatment device according to claim 1, wherein:

    - the light source is arranged to further emit optical radiation of a second wavelength,
    - the light sensor is arranged for providing separate CP and PP values for the first and the second wavelength, and

- the processor is further operative to combine the CP and PP values for the first and the second wavelength and to determine the MIP value based on the combined CP and PP values for the first and second wavelength.

12. A hair treatment device according to any one of the claims 1 to 11, further comprising a hair-cutting laser source and a further processor which is coupled to the light-based detector, wherein the further processor is arranged to activate the hair-cutting laser source in a position near the skin surface in which the light-based detector has detected the presence of a hair.

13. A method for detecting a hair near a skin surface, the method comprising:

- emitting optical radiation of at least a first wavelength and with an incident polarization towards the skin surface,
- detecting light reflected at the skin surface and having the incident polarization and providing a PP value representing the detected light with the incident polarization,
- detecting light reflected at the skin surface and having a different polarization and providing a CP value representing the detected light with the different polarization,
- based on a calibration procedure, scale the CP value and the PP value to respective dynamic ranges in order to obtain a scaled CP value and a scaled PP value respectively,
- determine a Minimum Intensity Projection (MIP) value of the set of the CP value and the PP value by selecting the lowest value of the scaled CP value and the corresponding scaled PP value, and
- discriminate between the skin surface and the hair based on the MIP value.

14. A computer program product for detecting a hair near a skin surface, which program is operative to cause a processor to perform the method as claimed in claim 13.

**Patentansprüche**

1. Haarbehandlungsvorrichtung mit einem lichtbasierten Detektor (10) zum Detektieren eines Haares nahe einer Hautoberfläche, wobei der lichtbasierte Detektor umfasst:

- eine Lichtquelle (13), um optische Strahlung mit zumindest einer ersten Wellenlänge und mit einer Einfallspolarisation zu der Hautoberfläche hin zu emittieren,
- einen Lichtsensor (14a, 14b), um an der Hautoberfläche reflektiertes Licht zu detektieren, wobei der Lichtsensor imstande ist, das reflektierte Licht mit der Einfallspolarisation und mit einer anderen Polarisation getrennt zu detektieren und einen das detektierte Licht mit der Einfallspolarisation darstellenden PP-Wert sowie einen das detektierte Licht mit der anderen Polarisation darstellenden CP-Wert vorzusehen, sowie
- einen Prozessor (15), der mit dem Lichtsensor gekoppelt ist und so arbeitet, dass er

- basierend auf einem Kalibriervorgang den CP-Wert und den PP-Wert auf jeweilige Dynamikbereiche skaliert, um einen skalierten CP-Wert beziehungsweise einen skalierten PP-Wert zu erhalten,
- durch Auswählen des niedrigsten Wertes des skalierten CP-Wertes und des entsprechenden skalierten PP-Wertes einen Minimumintensitätsprojektions-(MIP-) Wert des Satzes aus dem CP-Wert und dem PP-Wert ermittelt, und
- je nachdem, ob es sich bei dem ausgewählten MIP-Wert um den CP-Wert oder den PP-Wert handelt, zwischen der Hautoberfläche und dem Haar unterscheidet.

2. Haarbehandlungsvorrichtung nach Anspruch 1, wobei auf der Grundlage des Kalibriervorgangs, der eine Hintergrundmessung und einen diffusen Standard beinhaltet, die jeweiligen Dynamikbereiche ermittelt werden.

3. Haarbehandlungsvorrichtung nach Anspruch 1, wobei der Dynamikbereich für den PP-Wert in etwa dreimal so breit wie der Dynamikbereich für den CP-Wert ist.

4. Haarbehandlungsvorrichtung nach Anspruch 1, wobei der Prozessor weiterhin so arbeitet, dass er ein Verhältnis des skalierten CP-Wertes und des skalierten PP-Wertes berechnet und, weiter basierend auf dem berechneten Verhältnis, zwischen der Hautoberfläche und dem Haar unterscheidet.

5. Haarbehandlungsvorrichtung nach Anspruch 4, wobei der Prozessor weiterhin so arbeitet, dass er eine Unterscheidungsfunktion zur weiteren Verarbeitung des berechneten Verhältnisses anwendet, wobei die Unterscheidungsfunktion eine S-förmige Funktion mit einem steilen Abfall ist.

**6.** Haarbehandlungsvorrichtung nach Anspruch 5, wobei die Unterscheidungsfunktion einen Hyperbeltangens des berechneten Verhältnisses oder einer Linearfunktion des berechneten Verhältnisses umfasst.

**7.** Haarbehandlungsvorrichtung nach Anspruch 1, wobei der lichtbasierte Detektor weiterhin eine Steuereinheit umfasst, um die Lichtquelle so zu steuern, dass sie die optische Strahlung an mehreren Stellen in der Nähe der Hautoberfläche und an jeder Stelle, an der das reflektierte Licht unter Verwendung des Lichtsensors detektiert wird, emittiert, wobei der Prozessor weiterhin so arbeitet, dass er eine MIP-Darstellung vorsieht, die für jede Position den entsprechenden MIP-Wert anzeigt.

**8.** Haarbehandlungsvorrichtung nach Anspruch 7, wobei die mehreren Positionen eine eindimensionale Scan-Linie bilden.

**9.** Haarbehandlungsvorrichtung nach Anspruch 8, wobei mehrere eindimensionale Scan-Linien zusammen eine 2D-Scan-Oberfläche bilden.

**10.** Haarbehandlungsvorrichtung nach Anspruch 7, wobei der Prozessor weiterhin so arbeitet, dass er

- eine Verhältnisdarstellung berechnet, indem er für jede Position ein Verhältnis des entsprechenden CP-Wertes zu dem PP-Wert ermittelt,
- ein Gradientenfilter auf die Verhältnisdarstellung anwendet, um eine Randwahrscheinlichkeitsdarstellung zu erhalten, die für jede Position eine Wahrscheinlichkeit des Vorhandenseins eines Randes des Haares anzeigt, und
- die Randwahrscheinlichkeitsdarstellung und die MIP-Darstellung kombiniert, um eine Binärdarstellung zu erzeugen, die anzeigt, wo Haare zu erwarten sind.

**11.** Haarbehandlungsvorrichtung nach Anspruch 1, wobei:

- die Lichtquelle so eingerichtet ist, dass sie weiterhin optische Strahlung mit einer zweiten Wellenlänge emittiert,
- der Lichtsensor so eingerichtet ist, dass er für die erste und die zweite Wellenlänge einen getrennten CP- und PP-Wert vorsieht, und
- der Prozessor weiterhin so arbeitet, dass er den CP- und PP-Wert für die erste und die zweite Wellenlänge kombiniert und auf der Basis des kombinierten CP- und PP-Wertes für die erste und zweite Wellenlänge den MIP-Wert ermittelt.

**12.** Haarbehandlungsvorrichtung nach einem der Ansprüche 1 bis 11, die weiterhin eine Haarschneide-Laserquelle sowie einen weiteren Prozessor umfasst, der mit dem lichtbasierten Detektor gekoppelt ist, wobei der weitere Prozessor so eingerichtet ist, dass er die Haarschneide-Laserquelle in einer Position nahe der Hautoberfläche, in welcher der lichtbasierte Detektor das Vorhandensein eines Haares detektiert hat, aktiviert.

**13.** Verfahren zum Detektieren eines Haares nahe einer Hautoberfläche, wobei das Verfahren die folgenden Schritte umfasst, wonach:

- optische Strahlung mit zumindest einer ersten Wellenlänge und mit einer Einfallspolarisation zu der Hautoberfläche hin emittiert wird,
- an der Hautoberfläche reflektiertes Licht mit der Einfallspolarisation detektiert und ein PP-Wert vorgesehen wird, der das detektierte Licht mit der Einfallspolarisation darstellt,
- an der Hautoberfläche reflektiertes Licht mit einer anderen Einfallspolarisation detektiert und ein CP-Wert vorgesehen wird, der das detektierte Licht mit der anderen Polarisation darstellt,
- basierend auf einem Kalibriervorgang der CP-Wert und der PP-Wert auf jeweilige Dynamikbereiche skaliert werden, um einen skalierten CP-Wert beziehungsweise einen skalierten PP-Wert zu erhalten,
- durch Auswählen des niedrigsten Wertes des skalierten CP-Wertes und des entsprechenden skalierten PP-Wertes ein Minimumintensitätsprojektions-(MIP-) Wert des Satzes aus dem CP-Wert und dem PP-Wert ermittelt wird, und
- je nachdem, ob es sich bei dem ausgewählten MIP-Wert um den CP-Wert oder den PP-Wert handelt, zwischen der Hautoberfläche und dem Haar unterschieden wird.

**14.** Computerprogrammprodukt zum Detektieren eines Haares nahe einer Hautoberfläche, wobei das Programm so arbeitet, dass es bewirkt, dass ein Prozessor das Verfahren nach Anspruch 13 durchführt.

## Revendications

**1.** Dispositif de traitement de poils, comprenant un détecteur à base de lumière (10) pour détecter un poil à proximité d'une surface de la peau, le détecteur à base de lumière comprenant :

- une source lumineuse (13) pour émettre un rayonnement optique d'au moins une première longueur d'onde et avec une polarisation incidente vers la surface de la peau,
- un détecteur de lumière (14a, 14b) pour détecter une lumière réfléchie à la surface de la peau, le détecteur de lumière étant capable de détecter séparément la lumière réfléchie avec la polarisation incidente et avec une polarisation différente, et pour fournir une valeur PP représentant la lumière détectée avec la polarisation incidente et une valeur CP représentant la lumière détectée avec la polarisation différente, et
- un processeur (15) couplé au détecteur de lumière et opérationnel pour

- sur la base d'une procédure d'étalonnage, mettre la valeur CP et la valeur PP à l'échelle dans des plages dynamiques respectives afin d'obtenir respectivement une valeur CP mise à l'échelle et une valeur PP mise à l'échelle,
- déterminer une valeur de projection d'intensité minimale, MIP, de l'ensemble de la valeur CP et de la valeur PP en sélectionnant la plus petite valeur de la valeur CP mise à l'échelle et de la valeur PP mise à l'échelle correspondante, et
- discriminer entre la surface de la peau et le poil selon que la valeur MIP sélectionnée est la valeur CP ou la valeur PP.

**2.** Dispositif de traitement de poils selon la revendication 1, dans lequel les plages dynamiques respectives sont déterminées sur la base d'une procédure d'étalonnage impliquant une mesure contextuelle et un étalon diffus.

**3.** Dispositif de traitement de poils selon la revendication 1, dans lequel la plage dynamique pour la valeur PP est approximativement trois fois plus large que la plage dynamique pour la valeur CP.

**4.** Dispositif de traitement de poils selon la revendication 1, dans lequel le processeur est en outre opérationnel pour calculer un rapport de la valeur CP mise à l'échelle et de la valeur PP mise à l'échelle et pour discriminer entre la surface de la peau et le poil en outre sur la base du rapport calculé.

**5.** Dispositif de traitement de poils selon la revendication 4, dans lequel le processeur est en outre opérationnel pour utiliser une fonction de discrimination pour traiter en outre le rapport calculé, la fonction de discrimination étant une fonction en forme de S avec une forte pente.

**6.** Dispositif de traitement de poils selon la revendication 5, dans lequel la fonction de discrimination comprend une tangente hyperbolique du rapport calculé ou d'une fonction linéaire du rapport calculé.

**7.** Dispositif de traitement de poils selon la revendication 1, dans lequel le détecteur à base de lumière comprend en outre une unité de commande pour commander à la source lumineuse d'émettre le rayonnement optique à de multiples positions à proximité de la surface de la peau et, à chaque position, détecter la lumière réfléchie en utilisant le détecteur de lumière, le processeur étant en outre opérationnel pour fournir une cartographie de MIP indiquant, pour chaque position, la valeur MIP correspondante.

**8.** Dispositif de traitement de poils selon la revendication 7, dans lequel les multiples positions constituent une ligne de balayage unidimensionnel.

**9.** Dispositif de traitement de poils selon la revendication 8, dans lequel plusieurs lignes de balayage unidimensionnel constituent ensemble une surface de balayage bidimensionnel.

**10.** Dispositif de traitement de poils selon la revendication 7, dans lequel le processeur est en outre opérationnel pour :

- calculer une cartographie de rapport en déterminant, pour chaque position, un rapport de la valeur CP correspondante sur la valeur PP,

- appliquer un filtre de gradient à la cartographie de rapport afin d'obtenir une cartographie de probabilité de bord indiquant, pour chaque position, une probabilité de la présence d'un bord du poil, et
- combiner la cartographie de probabilité de bord et la cartographie de MIP pour générer une cartographie binaire indiquant où des poils sont censés se trouver.

11. Dispositif de traitement de poils selon la revendication 1, dans lequel

- la source lumineuse est agencée pour en outre émettre un rayonnement optique d'une deuxième longueur d'onde,
- le détecteur de lumière est agencé pour fournir des valeurs CP et PP distinctes pour la première longueur d'onde et la deuxième longueur d'onde, et
- le processeur est en outre opérationnel pour combiner les valeurs CP et PP pour la première longueur d'onde et la deuxième longueur d'onde et pour déterminer la valeur MIP sur la base des valeurs CP et PP combinées pour la première longueur d'onde et la deuxième longueur d'onde.

12. Dispositif de traitement de poils selon l'une quelconque des revendications 1 à 11, comprenant en outre une source laser de coupe de poils et un autre processeur qui est couplé au détecteur à base de lumière, dans lequel l'autre processeur est agencé pour activer la source laser de coupe de poils à une position à proximité de la surface de la peau à laquelle le détecteur à base de lumière a détecté la présence d'un poil.

13. Procédé de détection d'un poil à proximité d'une surface de la peau, le procédé comprenant :

- l'émission d'un rayonnement optique d'au moins une première longueur d'onde et avec une polarisation incidente vers la surface de la peau,
- la détection d'une lumière réfléchie à la surface de la peau et ayant la polarisation incidente et la fourniture d'une valeur PP représentant la lumière détectée avec la polarisation incidente,
- la détection d'une lumière réfléchie à la surface de la peau et ayant une polarisation différente et la fourniture d'une valeur CP représentant la lumière détectée avec la polarisation différente,
- sur la base d'une procédure d'étalonnage, la mise à l'échelle de la valeur CP et de la valeur PP dans des plages dynamiques respectives afin d'obtenir respectivement une valeur CP mise à l'échelle et une valeur PP mise à l'échelle,
- la détermination d'une valeur de projection d'intensité minimale, MIP, de l'ensemble de la valeur CP et de la valeur PP en sélectionnant la plus petite valeur de la valeur CP mise à l'échelle et de la valeur PP mise à l'échelle correspondante, et
- la discrimination entre la surface de la peau et le poil selon que la valeur MIP sélectionnée est la valeur CP ou la valeur PP.

14. Produit de programme informatique pour détecter un poil à proximité d'une surface de la peau, lequel programme est opérationnel pour amener un processeur à effectuer le procédé selon la revendication 13.

**Fig. 1**

**Fig. 2**

**Fig. 3**

**Fig. 4**

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010106480 A1
- WO 2010106480 A
- WO 00062700 A
- WO 2005099607 A
- WO 2010143108 A